# EUROPEAN PATENT APPLICATION

(11) **EP 2 898 792 A1**
(43) Date of publication of application: **29.07.2015**
(21) Application number: 15152310.7
(22) Date of filing: 23.01.2015
(51) Int. Cl.: A47B 21/03, A47B 39/10

(54) **Apparatus for correcting of user's posture attachable to a desk**

(30) Priority: 24.01.2014 KR 20140008788
(71) Applicant: Gmax Co., Ltd., Gwangju 500-896 (KR)
(72) Inventor: Yang, Don Seung, 500-896 Gwangju (KR); Oh, Byung Yong, 702-700 Daegu (KR)
(74) Representative: Gulde & Partner

(57) **Abstract**

An apparatus (1000) for correcting a posture attachable to a desk. The apparatus (1000) includes a desk fixing member (100) of which a side surface has a "C"-shape so as to be fixed on an upper plate of the desk and in which an inserting groove is formed along a vertical direction on a rear surface, an attaching member (50) including a desk attaching unit (54) which penetrates an upper surface or a lower surface of the desk fixing member (100) and is attached on the upper plate of the desk, and an attachment-adjusting screw (52) which adjusts a strength of attachment, a height adjusting member (200), a posture correcting pad (400), and a rotatable pad connecting member (300).

## Description

### [Technical Field]

The present invention relates to an assistant tool for study and office work, and more particularly, to an assistant tool capable of enhancing learning or working efficiency by reducing a user's physical fatigue by allowing a learner or a worker to maintain a good posture when learning or working in front of a desk.

### [Background Art]

Recently, various physical problems of students or office workers due to their bad posture habits have become an issue. Further, bad posture causes various kinds of fatigues, thereby ultimately weakening learning or working efficiency, and thus maintaining the correct posture in front of the desk may be very important.

In consideration of such importance of posture, various functional chair products and various pad products which are placed between a desk and a user's belly and help a user to have a good posture are sold in the market. However, such products are not adjustable according to the user's posture inclination and have limits in the actual effects as general-purpose products.

### [Disclosure]

### [Technical Problem]

An object of the present invention is to provide an apparatus for correcting a posture attachable to a desk, which may be variable and be optimized according to a user's posture characteristics or habits.

### [Technical Solution]

In order to solve the above problem, according to an aspect of the present invention, there is provided an apparatus for correcting a posture attachable to a desk, including a desk fixing member of which a side surface has a "C"-shape so as to be fixed on an upper plate of the desk and in which an inserting groove is formed along a vertical direction on a rear surface, an attaching member including a desk attaching unit which penetrates an upper surface or a lower surface of the desk fixing member and is attached on the upper plate of the desk, and an attachment-adjusting screw which adjusts a strength of attachment, a height adjusting member which is inserted into the inserting groove, of which the height may be adjusted by adjusting an inserting length, and which includes a rotation axis and a rotation locking unit at an end part thereof, a posture correcting pad which adjusts a user's posture located on a front surface of the desk and is attached to the user, and a rotatable pad connecting member of which one end is coupled with the posture correcting pad and the other end is coupled with a rotation axis of the height adjusting member so that the posture correcting pad may be connected to the height adjusting member, wherein the posture correcting pad is coupled to be rotatable through a coupling between the pad connecting member and a rotation axis, and wherein the posture correcting pad is rotatable by 180 or more degrees.

A height fixing member for fixing a modified height of the height adjusting member may be coupled with an external surface of the inserting groove.

The height fixing member may include a screw, and a penetration hole including a nut structure which the screw may penetrate may be formed on the external surface.

The posture correcting pad may include a pad supporting unit and a buffer pad which is coupled with the pad supporting unit and is made of elastic materials.

### [Advantageous Effects]

According to an apparatus for correcting a posture attachable to a desk according to an embodiment of the present invention, the height of a pad and the position of rotation may be variably changed, thus various combinations of the pad positions are possible, thus the user-tailored various modifications are possible, thereby being generally usable to all users having various postures.

Particularly, the present invention has an advantage of adjusting the distance between the user's front part and the pad as well as the height because the pad for correcting the posture includes a height-changing element and two rotatable elements.

### [Description of Drawings]

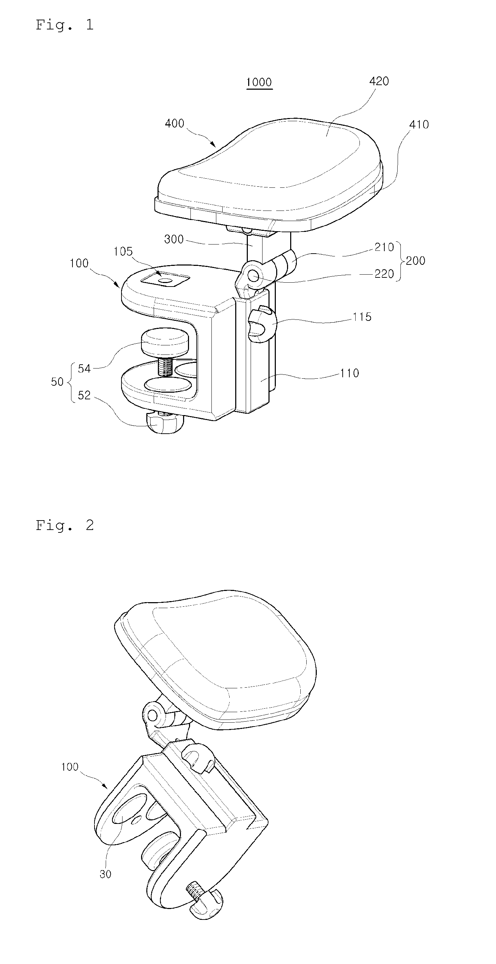

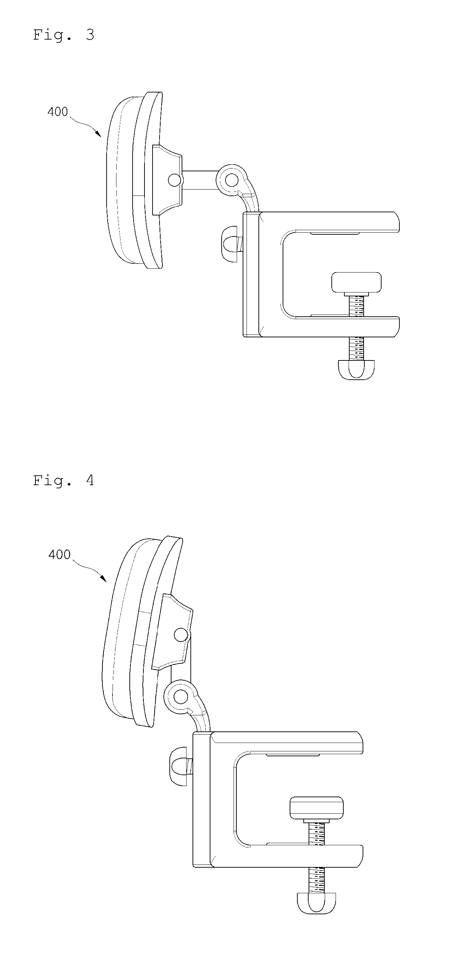

FIG. 1 is a perspective view of an apparatus for correcting a posture attachable to a desk according to an embodiment of the present invention. FIG. 2 is a perspective view of a desk protecting member of the apparatus for correcting the posture attachable to the desk of FIG. 1. FIG. 3 illustrates an example of utilizing a posture-correcting pad of FIG. 1. FIG.

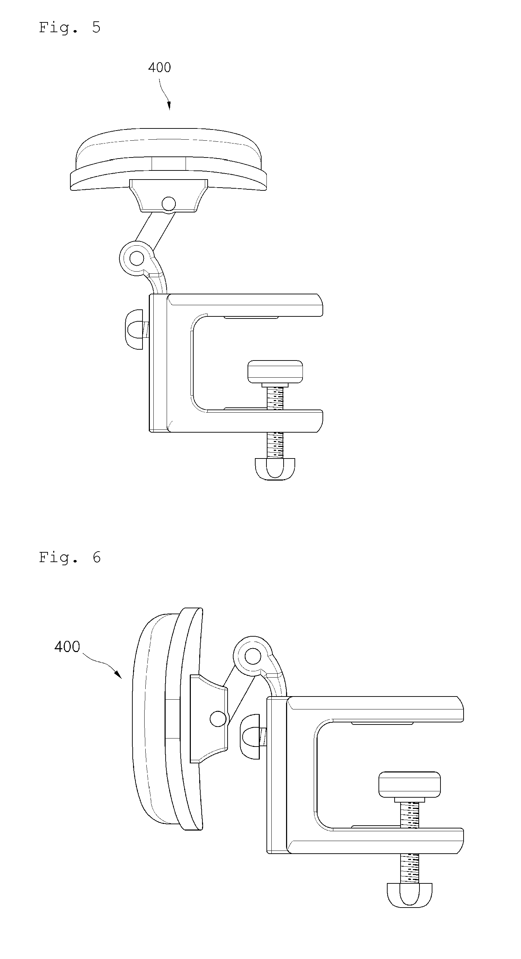

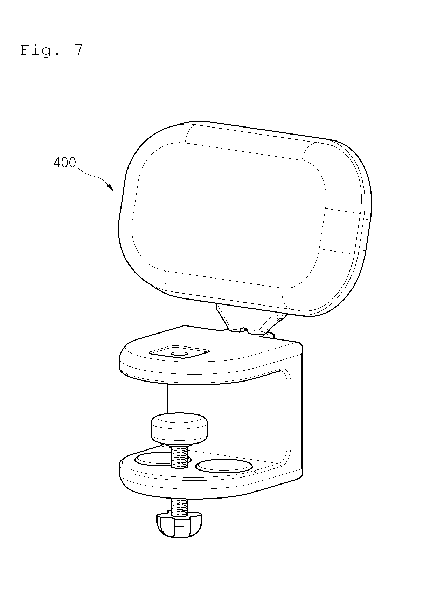

4 illustrates another example of utilizing the posture-correcting pad of FIG. 1. FIG. 5 illustrates further another example of utilizing the posture-correcting pad of FIG. 1. FIG. 6 illustrates further another example of utilizing the posture-correcting pad of FIG. 1. FIG. 7 illustrates further another example of utilizing the posture-correcting pad of FIG. 1.

### [Detailed Description of Exemplary Embodiments]

Hereinafter, an apparatus for correcting a posture attachable to a desk according to an embodiment of the present invention will be described in detail with reference to the attached drawings. However, the description below is merely illustrative and the technical concept of the present invention may be determined only by the claims to be described later.

FIG. 1 is a perspective view of an apparatus for correcting a posture attachable to a desk according to an embodiment of the present invention.

Referring to FIG. 1, an apparatus 1000 for correcting a posture attachable to a desk includes a desk fixing member 100, a height adjusting member 200, a pad connecting member 300, and a posture correcting pad 400.

The desk fixing member 100 performs a function of allowing the apparatus 100 for correcting the posture attachable to the desk to be attached on the desk. The desk fixing member 100 may have a "C" shape, and an upper plate of the desk having a certain thickness is inserted and fixed at the groove part of the "C" shape. Because the desk fixing member 100 have the "C" shape, the desk fixing member 100 includes an opened side surface, an upper surface, a lower surface, and a rear surface.

A penetration hole 105 is formed on the upper surface and the lower surface of the desk fixing member 100, and thus an attaching member may be arranged as shown in FIG. 1. The attaching member 50 includes an attaching unit 54 which is attached on the upper plate of the desk and an attachment-adjusting screw 52. The attachment-adjusting screw appropriately adjusts the attaching pressure between the attaching unit 54 and the desk surface while moving vertically. An inserting nut, which is formed by an inserting injection scheme, is formed at the inner wall of the penetration hole 105 which is formed on the upper surface and the lower surface. The attachment-adjusting screw 52 may be vertically moved by the inserting nut. The penetration hole 105 is formed at both the upper surface and the lower surface so that the attachment-adjusting screw 52 may be selectively fixed at both the upper surface and the lower surface, thereby becoming applicable to various desk environments. Further, though not illustrated, a nut structure, which may be coupled with the attachment-adjusting screw 52, may be formed at the lower part of the attaching unit 54. The surface of the attaching unit 54, which directly contacts the upper plate of the desk, may be made of ABS resin, etc.

An inserting groove 110 is formed at the rear surface of the desk fixing member 100 so that the above described height adjusting member 200 may be inserted and moved in the vertical direction. The inserting groove 110 has a structure that a part of the rear surface of the inserting groove 110 is projected to have a space where the desk fixing member 100 may be moved.

Referring to FIG. 1 again, the height adjusting member 200 includes a height adjusting body 210 which is inserted into the inserting groove 110 and a rotation locking unit 220 which is formed at the end part of the exposed portion of the height adjusting body 210. A rotation axis is included in the end part of the height adjusting body 210, and a rotation locking unit 220, which locks or opens the rotation of the rotation axis, is formed. Various known technologies may be utilized as such a rotation locking unit 220. For example, as a button type, when a button is pushed, the rotation axis may be set to be rotatable, and when the button is pushed again, the rotation axis may be fixed.

Though not illustrated in the attached drawings, a plurality of grooves or penetration holes may be formed in the height adjusting body 210 in order to allow height adjustment in a plurality of steps. Further, a penetration hole, where an inserting nut is formed, exists at the external surface of the inserting groove 100 which is formed at the rear surface of the desk fixing member 100, and as illustrated in FIG. 1, the height of the height adjusting body 210 may be adjusted by the height adjusting body member 115 including the screw structure so as to be fixed.

Referring to FIG. 1, the posture correcting pad 400 is connected to the height adjusting member 200 by the pad connecting member 300. Specifically, one end of the pad connecting member 300 is coupled with the posture correcting pad 400, and the other end is coupled with the rotation axis which is positioned on the same line as that of the rotation locking unit 200 of the height adjusting member 200 so as to connect the posture correcting pad to the height adjusting member 200. The pad connecting member 300 may also be connected with the posture correcting pad 400 through a rotation axis coupling so as to be rotatable. The position of the posture correcting pad 400 may be rotated by 180 or more degrees by rotation or fixing, and thereby various positions of the posture correcting pad 400 may be applied. Further, the distance between the user's front part and the pad as well as the height may also be adjusted by including a height modifying element and two rotatable elements of the posture correcting pad 400.

The posture correcting pad 400 includes a buffer pad 420 which is substantially attached, and a pad support unit 410 which supports the buffer pad 420. For example, the buffer pad 420 may be coupled by an adhesive, etc. after being inserted into a groove (not shown) which is formed at the front surface of the pad support unit 410.

FIG. 2 is a perspective view of a desk protecting member of the apparatus for correcting the posture attachable to the desk of FIG. 1.

Referring to FIG. 2, at least one desk protecting member 30 is formed at one area of the inner surface of the upper and lower surfaces of the desk fixing member 100. The shape of the desk protecting member 30 is not particularly limited. PVC resin, etc. may be used as the material of the desk protecting member 30. The desk protecting member 30 is formed to prevent the damage of the upper plate of the desk which is inserted into and fixed at the desk fixing member 100.

FIG. 3 illustrates an example of utilizing a posture-correcting pad of FIG. 1. FIG. 4 illustrates another example of utilizing the posture-correcting pad of FIG. 1. FIG. 5 illustrates further another example of utilizing the posture-correcting pad of FIG. 1. FIG. 6 illustrates further another example of utilizing the posture-correcting pad of FIG. 1. FIG. 7 illustrates further another example of utilizing the posture-correcting pad of FIG. 1.

That is, FIGS. 3 to 7 illustrate position changes of various posture correcting pads 400 which may be utilized by using the above-described height adjusting unit or the pad rotation unit, etc.

Referring to FIG. 3, the arrangement relation of the posture correcting pad 400, which is vertical to the user's front at a specific height, is illustrated, and there may be the maximum distance between the user and the desk.

FIG. 4 illustrates the arrangement relation in which the rotation of the posture correcting pad 400 is changed so that the posture correcting pad 400 has the minimum distance with the user at a certain height and the height becomes the maximum. This shows that the posture correcting pad 400 may be closely attached up to the chest area depending on the user's preference.

FIG. 5 illustrates an appearance in which the position relation of the posture correcting pad 400 is modified so that the posture correcting pad 400 may be laid in a direction parallel to the surface of the desk at a certain height. This is an example of utilizing the posture correcting pad 400 which allows the upper part of the user to be attached when the user lies.

FIG. 6 illustrates a position relation in which the posture correcting pad 400 is closely attached to the average waist area with the minimum distance with the user.

FIG. 7 illustrates an appearance in which the posture correcting pad 400 and the user face opposite surfaces. For example, this illustrates the arrangement relation of the posture correcting pad 400, which may be considered when the posture correcting apparatus is not being used, i.e., at the time of storage, etc.

Likewise, various user-tailored utilizations according to various arrangements of the posture correcting pad 400 are possible.

**[Description of Reference Numerals]**

| | | | |
|---|---|---|---|
| 30: | desk protecting member | 50: | attaching member |
| 52: | attachment-adjusting screw | 54: | desk attaching unit |
| 100: | desk fixing member | 105: | penetration hole |
| 110: | inserting groove | 115: | height fixing member |
| 200: | height adjusting member | 210: | height adjusting body |
| 220: | rotation fixing unit | 300: | pad connecting member |
| 400: | posture correcting pad | 410: | pad supporting unit |
| 420: | buffer pad | | |
| 1000: | apparatus for correcting posture attachable to desk | | |

## Claims

1. An apparatus for correcting a posture attachable to a desk, the apparatus comprising:
a desk fixing member of which a side surface has a "C"-shape so as to be fixed on an upper plate of the desk and in which an inserting groove is formed along a vertical direction on a rear surface;
an attaching member including a desk attaching unit which penetrates an upper surface or a lower surface of the desk fixing member and is attached on the upper plate of the desk, and an attachment-adjusting screw which adjusts a strength of attachment;
a height adjusting member which is inserted into the inserting groove, of which the height may be adjusted by adjusting an inserting length, and which includes a rotation axis and a rotation locking unit at an end part thereof;
a posture correcting pad which adjusts a user's posture located on a front surface of the desk and is attached to the user; and
a rotatable pad connecting member of which one end is coupled with the posture correcting pad and the other end is coupled with a rotation axis of the height adjusting member so that the posture correcting pad may be connected to the height adjusting member,
wherein the posture correcting pad is coupled to be rotatable through a coupling between the pad connecting member and a rotation axis, and
wherein the posture correcting pad is rotatable by 180 or more degrees.

2. The apparatus of claim 1, wherein a height fixing member for fixing a modified height of the height adjusting member is coupled with an external surface of the inserting groove.

3. The apparatus of claim 2, wherein the height fixing member includes a screw, and a penetration hole including a nut structure which the screw may penetrate is formed on the external surface.

4. The apparatus of claim 1, wherein the posture correcting pad includes a pad supporting unit and a buffer pad which is coupled with the pad supporting unit and is made of elastic materials.
